(19) Europäisches Patentamt
European Patent Office
Office européen des brevets

(11) Publication number: 0 533 925 A1

# (12) EUROPEAN PATENT APPLICATION published in accordance with Art. 158(3) EPC

(21) Application number: 91909702.2

(22) Date of filing: 28.05.91

(86) International application number: PCT/JP91/00714

(87) International publication number: WO 91/19694 (26.12.91 91/29)

(51) Int. Cl.[5]: C07C 255/54, C07C 253/02, C07C 253/30, C07D 213/643, A01N 39/02, A01N 43/40

(30) Priority: 11.06.90 JP 149865/90

(43) Date of publication of application: 31.03.93 Bulletin 93/13

(84) Designated Contracting States: BE CH DE DK ES FR GB GR IT LI NL SE

(71) Applicant: TEIJIN LIMITED
6-7, Minamihonmachi 1-chome Chuo-ku
Osaka-shi Osaka 541(JP)

(72) Inventor: AZUMA, Shizuo
4-22-1, Hirata, Iwakuni-shi
Yamaguchi 741(JP)
Inventor: HIRAMATSU, Toshiyuki
1981-9, Tsuzu, Iwakuni-shi
Yamaguchi 740(JP)
Inventor: ICHIKAWA, Yataro
2-11-7, Kotesashi-cho, Tokorozawa-shi
Saitama 359(JP)

(74) Representative: Votier, Sidney David et al
CARPMAELS & RANSFORD 43, Bloomsbury Square
London WC1A 2RA (GB)

## (54) OPTICAL ISOMER OF R-FORM OF DIPHENYL ETHER DERIVATIVE AND HERBICIDE CONTAINING THE SAME AS ACTIVE INGREDIENT.

(57) A herbicide containing an optical isomer of R-form of a diphenyl ether derivative represented by formula (I), which has a more excellent herbicidal activity with a smaller dose thereof than one containing the corresponding racemate. The use of the compound of formula (I) together with various herbicides known *per se* serves to improve the fast-acting property and widen the herbicidal spectrum with a small dose.

EP 0 533 925 A1

Field of the Invention:

The present invention relates to R-enantiomers of diphenyl ether and herbicidal compositions containing the same as an active ingredient. Particularly, the present invention relates to a herbicide which shows excellent activity against broad leaved and narrow leaved weeds, and has selective herbicidal activity without any adverse effect on the growth of crop plants by choosing the application methods, treating methods and application rates appropriately.

Description of the Prior Arts:

Herbicides of the type which selectively kill broad-leaved weeds, typified by 2,4-dichlorophenoxyacetic acid, are known as compounds of herbicidal selectivity. The selectivity of the herbicidal activity of 2,4-dichlorophenoxyacetic acid is between narrow-leaved plants including both crop plants and weeds, and broad-leaved plants including both crop plants and weeds. It is known that 2,4-dichlorophenoxyacetic acid has very little or no activity against narrow-leaved plants [see, for example, Nature, 155, 498 (1945)]. It is known, on the other hand, that compounds resulting from introduction of a chlorine- or trifluoromethyl-substituted phenoxy group or a chlorine- or trifluoromethyl-substituted pyridyloxy group into the aromatic group of the above-stated compounds have the activity of selectively killing narrow-leaved plants (see U.S. Patents Nos. 4,270,948; 4,309,562; 4,314,069; 4,332,961 and 3,954, 442; British Patent No. 1,579,201, and Japanese Laid-Open Patent Publication Nos. 125626/1977 and 15825/1977). These compounds, however, also kill useful crops such as rice or corn.

A racemic methyl ester of α-[2-cyano-5-(2-chloro-4-trifluoromethylphenoxy)phenoxy]propionic acid is used to control weeds (Japanese Laid-open Patent Publication No. 111528/1977), but the requirement of a large application rate is pointed out.

Further, herbicides containing a kind of N-phosphonomethylglycine derivative as an active ingredient are known and commercially available. The N-phosphonomethylglycine derivative is basically a non-selective herbicide, but its herbicidal activity is reduced in a low application rate against perennial weeds such as Cyperus rotundus in Cyperaceae and against broad-leaved weeds such as Chenopodium album var. centrorubrum or Amaranthus retroflexas. Particularly, it hardly manifests herbicidal activity against weeds in Convolvulaceae such as Ipomoea purpurea even 2 weeks after application. Further, the N-phosphonomethylglycine derivative is slow acting and unsuitable for the case that the weeds must be withered quickly and the next work should be started soon in crop lands or non-crop lands. For example, in a crop land, all of the weeds should be eradicated before sowing of a crop plant, but the slow acting may cause ad verse effects such as delay of sowing time and damage to the crop seeds. Additionally, in non-crop lands, delays in constructions of buildings or railroads or weeding delay in roads may be caused, thus the N-phosphonomethylglycine derivative is unsatisfactory as a non-selective herbicide with quick-action at a low application rate.

Herbicides containing some kinds of glufosinate com pounds as a major active ingredient are known and commercially available. This type of herbicide also is basically non-selective, but has a disadvantage that a low application rate reduces herbicidal activity against broad-leaved weeds such as Chenopondium album var. centrorubrum, Amaranthus retroflexas or Abutilon theophrasti.

The Objects of the Invention:

The inventors of this invention have made intensified study in order to remove these disadvantages and found that R-enantiomers of a specific diphenyl ether derivative exhibits excellent herbicidal activity with more reduced application rate, thus attaining the present invention.

Disclosure of the Invention:

Namely, the present invention is an R-enantiomer of diphenyl ether derivative represented by the following formula (I):

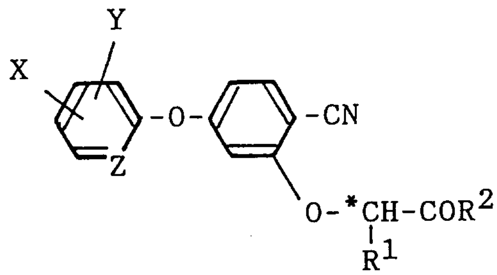

( I )

(R-enantiomer)

wherein
X and Y are identical or different and each represents a hydrogen atom, a halogen atom, $-CF_3$, or an alkyl group having 1 to 5 carbon atoms;
Z is =CH- or =N-;
$R^1$ is an alkyl group having 1 to 5 carbon atoms or a phenyl group;
$R^2$ is a hydroxy group, an alkyl group having 1 to 5 carbon atoms which may be optionally substituted with halogen atoms, a phenyl group which may be substituted with halogen atoms, an alkoxy group having 1 to 5 carbon atoms, an alkenyloxy group having 2 to 5 carbon atoms or

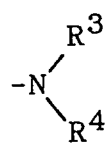

in which $R^3$ and $R^4$ are a hydrogen atom or an alkyl group having 1 to 5 carbon atoms;
C* represents an asymmetric carbon atom;
or its salt compound and a herbicidal composition containing said compound as a major active ingredient.

The present invention will be illustrated in detail.

In the above-described formula (I), X and Y are identical or different and each represents a hydrogen atom, a halogen atom, $-CF_3$ or an alkyl group having 1 to 5 carbon atoms.

The halogen atom means for example, fluorine, chlorine or bromine or the like. The alkyl group having 1 to 5 carbon atoms may be straight-chained or branched, means methyl, ethyl, n-propyl, iso-propyl, n-butyl, sec.-butyl, iso-butyl, t-butyl, or n-pentyl. X and Y are identical or different and preferably means chlorine or $-CF_3$.

In the above-described formula (I), Z is =CH- or =N-.

In the above-described formula (I), $R^1$ is an alkyl group having 1 to 5 carbon atoms or a phenyl group. As the alkyl group, can be cited the same one as cited in the definition of X and Y.

In the formula (I), $R^2$ is a hydroxy group, an alkyl group having 1 to 5 carbon atoms which may be optionally substituted with a halogen atom, a phenyl group which may be substituted with halogen atoms, an alkoxy group having 1 to 5 carbon atoms, an alkenyloxy group having 2 to 5 carbon atoms, or

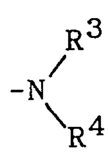

wherein $R^3$ and $R^4$ are a hydrogen atom or an alkyl group having 1 to 5 carbon atoms, respectively.
As the halogen atom and alkyl group described above, are cited the same ones as given in the definition of X and Y. The alkoxy group having 1 to 5 carbon atoms may be straight-chained or branched and is methoxy, ethoxy, n-propoxy, iso-propoxy, n-butoxy, sec.-butoxy, tert.-butoxy, n-pentyloxy or the like. The alkenyloxy group having 2 to 5 carbon atoms is ethenoxy, propenoxy, butenoxy, pentenoxy or the like.

The following compounds can be cited as R-enantiomer of the diphenyl ether derivative of formula (I):

(1)Methyl α-[2-cyano-5-(2-chloro-4-trifluoromethylphenoxy)phenoxy]propionate,

(2) Ethyl α-[2-cyano-5-(2-chloro-4-trifluoromethylphe noxy)phenoxy]propionate,
(3) n-Butyl α-[2-cyano-5-(2-chloro-4-trifluoromethylphenoxy)phenoxy]propionate,
(4) α-[2-cyano-5-(2-chloro-4-trifluoromethylphenoxy)phenoxy]propionic acid,
(5) Methyl α-[2-cyano-5-(2-chloro-4-trifluoromethylphenoxy)phenoxy]butyrate,
(6) Ethyl α-[2-cyano-5-(2-chloro-4-trifluoromethylphenoxy)phenoxy]butyrate,
(7) n-Butyl α-[2-cyano-5-(2-chloro-4-trifluoromethylphenoxy)phenoxy]butyrate,
(8) α-[2-cyano-5-(2-chloro-4-trifluoromethylphenoxy)phenoxy]butyric acid,
(9) Methyl α-[2-cyano-5-(2-chloro-4-trifluoromethylphenoxy)phenoxy]valerate,
(10) Ethyl α-[2-cyano-5-(2-chloro-4-trifluoromethylphe noxy)phenoxy]valerate,
(11) n-Butyl α-[2-cyano-5-(2-chloro-4-trifluoromethylphe noxy)phenoxy]valerate,
(12) α-[2-cyano-5-(2-chloro-4-trifluoromethylphenoxy)phenoxy]propionamide,
(13) N-Ethyl α-[2-cyano-5-(2-chloro-4-trifluoromethylphenoxy)phenoxy]propionamide,
(14) Allyl α-[2-cyano-5-(2-chloro-4-trifluoromethylphenoxy)phenoxy]propionate,
(15) Vinyl α-[2-cyano-5-(2-chloro-4-trifluoromethylphenoxy)phenoxy]propionate,
(16) Allyl α-[2-cyano-5-(2-chloro-4-trifluoromethylphenoxy)phenoxy]butyrate,
(17) Vinyl α-[2-cyano-5-(2-chloro-4-trifluoromethylphenoxy)phenoxy]butyrate,
(18) Allyl α-[2-cyano-5-(2-chloro-4-trifluoromethylphenoxy)phenoxy]valerate,
(19) Vinyl α-[2-cyano-5-(2-chloro-4-trifluoromethylphenoxy)phenoxy]valerate,
(20) N,N-Diethyl α-[2-cyano-5-(2-chloro-4-trifluoromethylphenoxy)phenoxy]propionamide,
(21) Methyl α-[2-cyano-5-(2,4-dichlorophenoxy)phenoxy]propionate,
(22) Ethyl α-[2-cyano-5-(2,4-dichlorophenoxy)phenoxy]propionate,
(23) n-Butyl α-[2-cyano-5-(2,4-dichlorophenoxy)phenoxy]propionate,
(24) Vinyl α-[2-cyano-5-(2,4-dichlorophenoxy)phenoxy]propionate,
(25) Allyl α-[2-cyano-5-(2,4-dichlorophenoxy)phenoxy]propionate,
(26) Methyl α-[2-cyano-5-(2,4-dichlorophenoxy)phenoxy]butyrate,
(27) Ethyl α-[2-cyano-5-(2,4-dichlorophenoxy)phenoxy]butyrate,
(28) Methyl α-[2-cyano-5-(2,4-dinitrophenoxy)phenoxy]propionate,
(29) Ethyl α-[2-cyano-5-(2,4-dinitrophenoxy)phenoxy]propionate,
(30) Methyl α-[2-cyano-5-(2-nitro-4-trifluoromethylphenoxy)phenoxy]propionate,
(31) Ethyl α-[2-cyano-5-(2-nitro-4-trifluoromethylphenoxy)phenoxy]propionate,
(32) Methyl α-[2-cyano-5-(2-cyano-4-trifluoromethylphenoxy)phenoxy]propionate,
(33) Ethyl α-[2-cyano-5-(2-cyano-4-trifluoromethylphenoxy)phenoxy]propionate,
(34) α-[2-cyano-5-(2-nitro-4-trifluoromethylphenoxy)phenoxy]propionic acid,
(35) α-[2-cyano-5-(2-cyano-4-trifluoromethylphenoxy)phenoxy]propionic acid,
(36) Methyl α-[2-cyano-5-(3-chloro-5-trifluoromethyl-2-pyridyloxy)phenoxy]propionate,
(37) Ethyl α-[2-cyano-5-(3-chloro-5-trifluoromethyl-2-pyridyloxy)phenoxy]propionate,
(38) n-Butyl α-[2-cyano-5-(3-chloro-5-trifluoromethyl-2-pyridyloxy)phenoxy]propionate,
(39) Methyl α-[2-cyano-5-(3-chloro-5-trifluoromethyl-2-pyridyloxy)phenoxy]butyrate,
(40) Ethyl α-[2-cyano-5-(3-chloro-5-trifluoromethyl-2-pyridyloxy)phenoxy]butyrate,
(41) n-Butyl α-[2-cyano-5-(3-chloro-5-trifluoromethyl-2-pyridyloxy)phenoxy]butyrate,
(42) Methyl α-[2-cyano-5-(3-chloro-5-trifluoromethyl-2-pyridyloxy)phenoxy]valerate,
(43) Ethyl α-[2-cyano-5-(3-chloro-5-trifluoromethyl-2-pyridyloxy)phenoxy]valerate,

The compounds of formula (I) can be produced, for example, by the processes shown in the following reaction schemes, (i), (ii), (iii), (iv), (v), and (vi). The processes are not limited only to them.

(i)

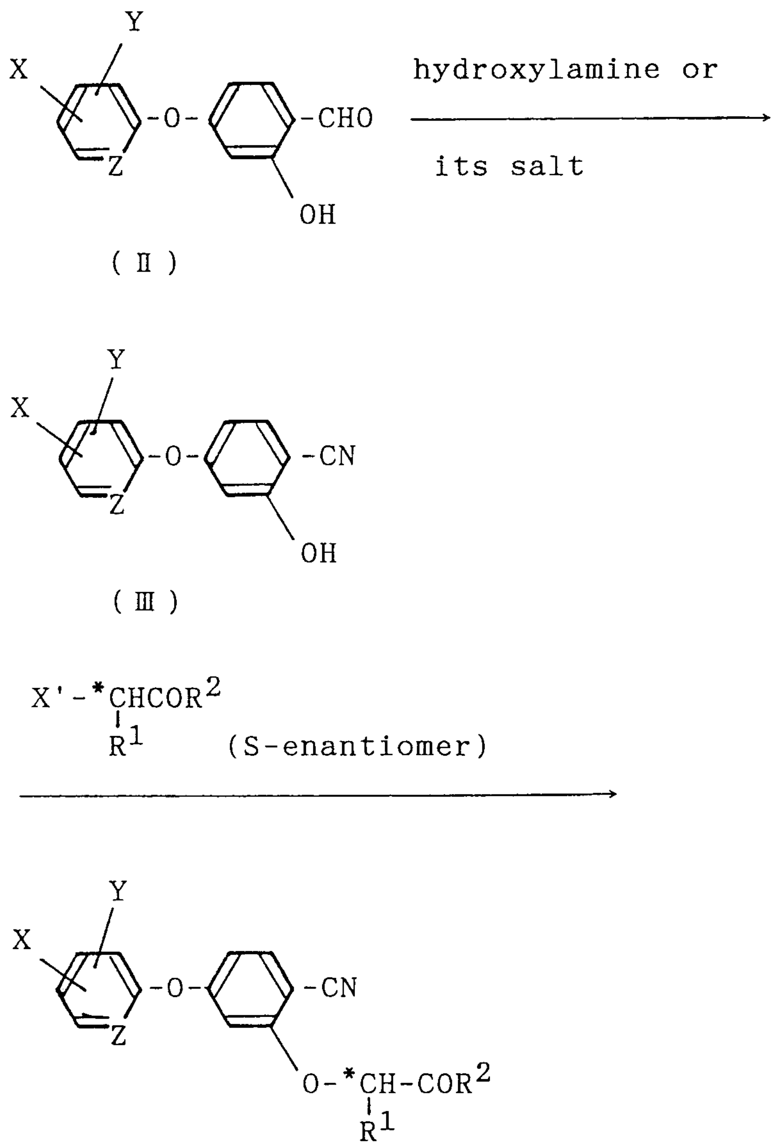


( II )

( III )

( I ) (R-enantiomer)

(ii)

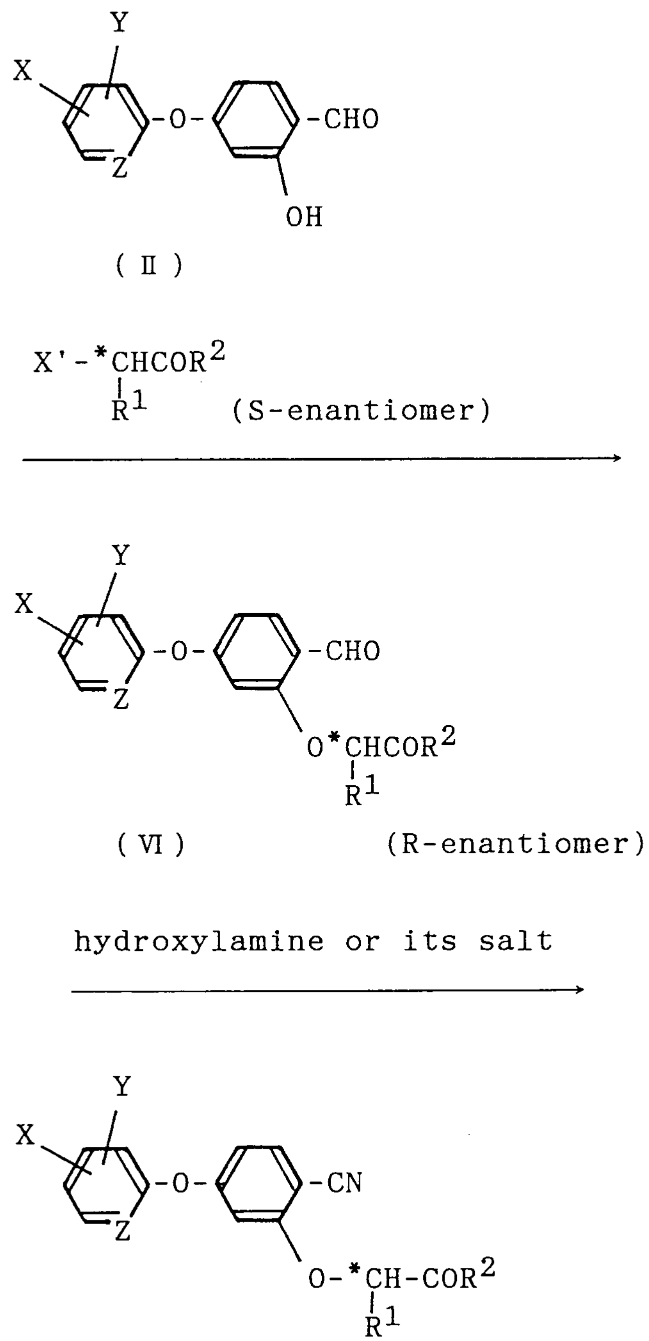


( II )

( VI ) (R-enantiomer)

( I ) (R-enantiomer)

(iii)

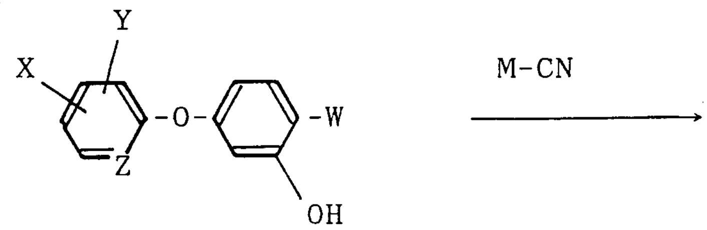


( VII )

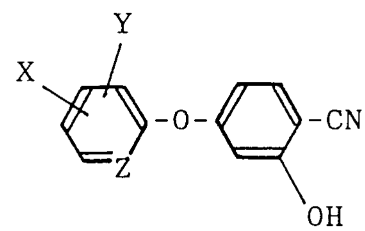

( III )

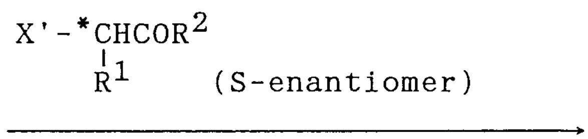


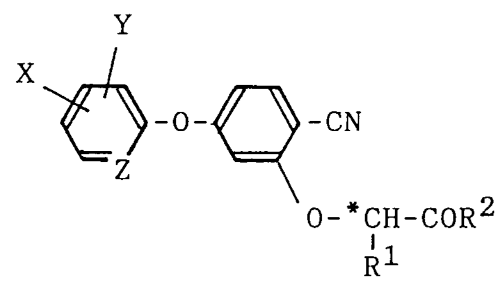

( I ) (R-enantiomer)

(iv)

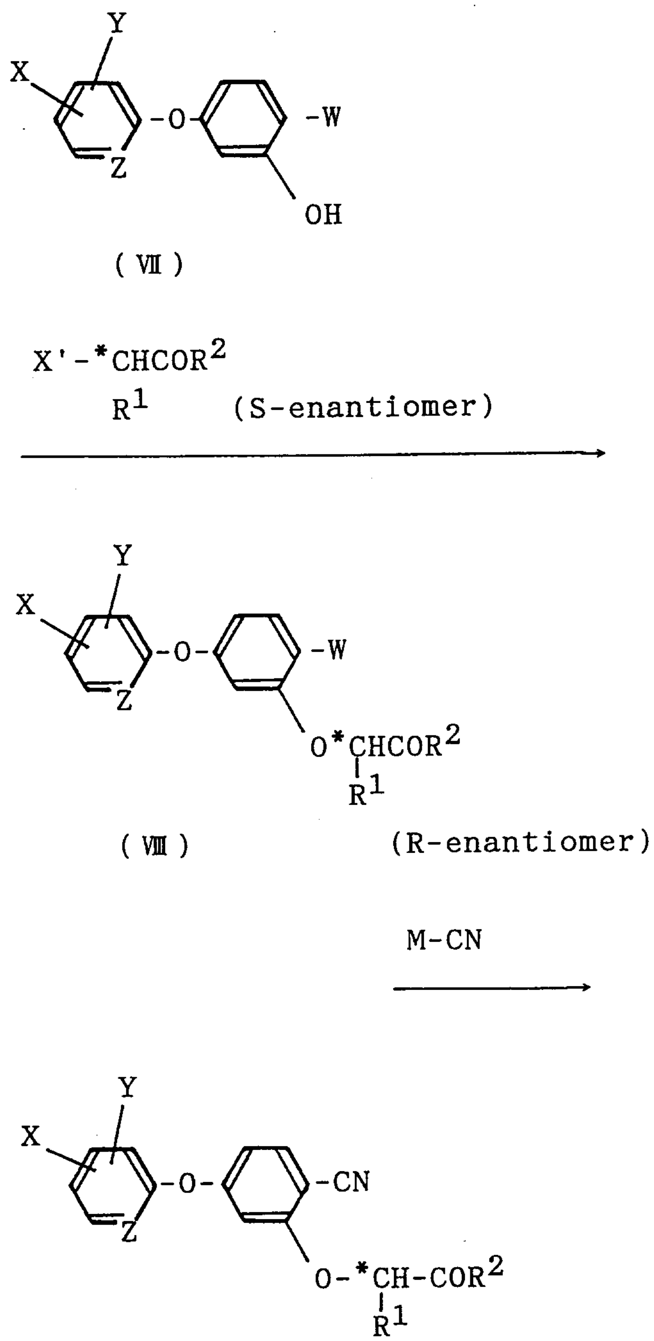


(VII)

(VIII)

(I) (R-enantiomer)

(v)

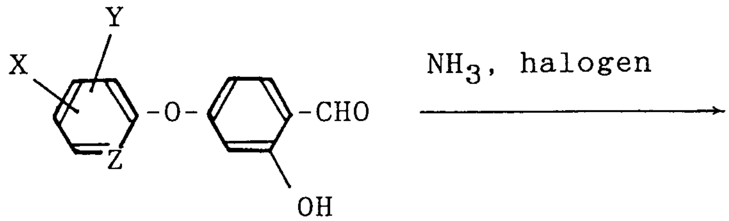


( II )

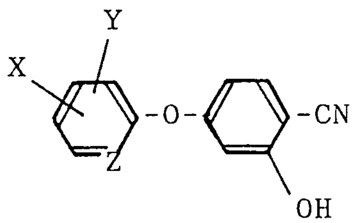

( III )

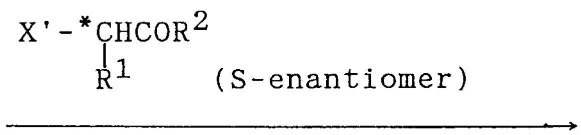


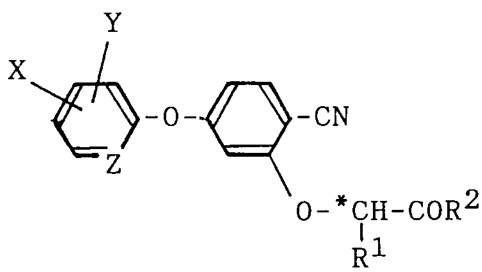

( I ) (R-enantiomer)

(vi)

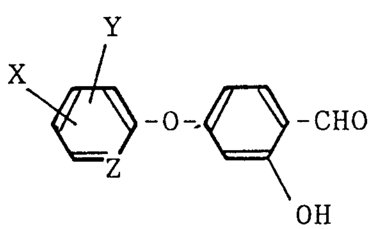

( II )

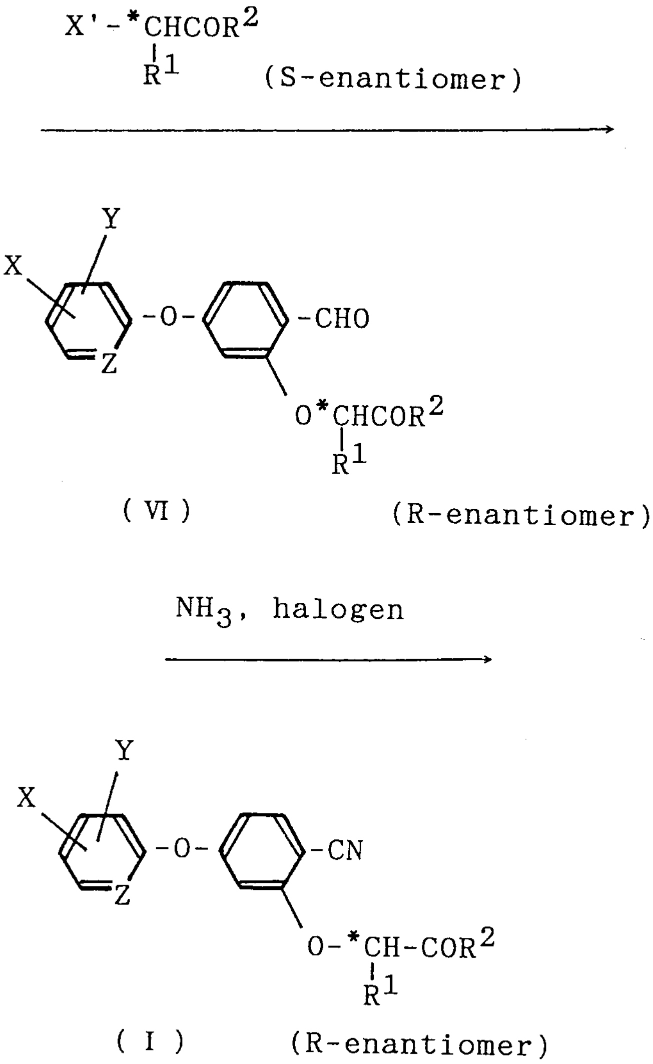


( VI )

( I )

wherein X, Y, Z, $R^1$, $R^2$ and *C are defined as in formula (I) and X' means a halogen atom or $-OR^5$; wherein $R^5$ is an alkylsulfonyl group or an arylsulfonyl group; W is a halogen atom, and M represents an alkali metal, copper, zinc, nickel or the like.

The hydroxylamine or its salt to be used in the processes (i) and (ii) stated above is hydroxylamine, hydroxylamine hydrochloride, hydroxylamine sulfate or the like. In these processes, an alkali metal or alkaline earth metal salt of an organic acid may be combined and the salt is, for example, sodium formate, potassium formate, calcium formate, magnesium formate, sodium acetate, potassium acetate, calcium acetate, magnesium acetate or the like. Further, sodium benzoate, potassium benzoate, calcium benzoate, magnesium benzoate or the like may employed, too. In some cases, an inorganic base such as sodium hydroxide, potassium hydroxide, sodium carbonate, or potassium carbonate and an organic base such as pyridine, triethylamine, diazabicycloundecene, triethylenediamine, or benzyltrimethylammonium hydroxide can be used, too. The solvent may be present or absent, and an organic carboxylic acid such as formic or acetic acid, an aromatic hydrocarbon such as benzene, toluene, xylene or the like, ethyl acetate, tetrahydrofuran, dioxane, ethyl ether, acetone, methyl ethyl ketone, dimethyl sulfoxide, N,N-dimethyl formamide, N-methylpyrrolidone, pyridine can be employed. Further, a solvent slightly compatible with water may be used in a two-phase system.

The reactions are usually carried out at 0 to 200 ° C, preferably at 30 to 150 ° C. When a solvent is used, whose boiling point is in the temperature range of the reaction, the reaction is effected at the boiling point in many cases.

M-CN to be used in the processes (iii) and (iv) includes an alkali metal, alkaline earth metal, copper, zinc or the like as M. W is a halogen atom, preferably chlorine or bromine. The reaction temperature is usually 0 to 150 ° C, preferably from about room temperature to 100 ° C.

As an oxidizing agent to be used in the processes (v) and (vi), are cited, for example, a halogen such as bromine or iodine, lead tetraacetate or cupric chloride. In some cases, an ammine cobalt complex can be used instead of the oxidant cited above and ammonia. Any solvent can be employed as long as it can substantially dissolve the aldehyde, ammonia and other starting materials. In some cases, a base is used and an inorganic base such as sodium hydroxide, potassium hydroxide, sodium carbonate or potassium carbonate, or an alcoholate such as sodium methoxide, sodium ethoxide or potassium tert.-butoxide can exemplified. The reaction temperature is 0 to 100 ° C, preferably in the rage from 10 to 80 ° C.

The R-enantiomers of diphenyl ether derivatives of formula (I) according to the present invention have the property of affecting the metabolism of plants, for example, to inhibit the growth of a certain kind of plants, regulate the growth of a certain kind of plants, dwarf a certain kind of plants or kill a certain kind of plants.

The compounds of formula (I) according to the present invention can be applied to seeds of plants, as well as to plants in various growth stages through foliage and/or roots. In other words, the compounds provided by the present invention are applied, either as such or in the form of a composition, to the plants whose growth is to be inhibited, namely whose metabolism is to be regulated, to seeds of such plants, to a locus where such plants are growing or a locus where such plants are expected to grow in an amount sufficient to control the metabolism of such plants.

The compounds according to the present invention can control the metabolism of plants at a rate of, for example, 0.001 to 20 kg/ha, preferably 0.005 to 10 kg/ha, particularly 0.01 to 5 kg/ha.

The compounds according to the present invention can be used in usual formulation such as a solution, an emulsifiable concentrate, a suspension, a dust, a paste or granules.

Such formulation can be prepared by using at least one or more agriculturally acceptable dilutent, for example, solid carriers such as talc bentonite, clay, kaolin, diatomaceous earth, white carbon, vermiculite, slaked lime, ammonium sulfate and urea; liquid carriers such as water, alcohols, dioxane, acetone, xylene, cyclohexane, methylnaphthalene, dimethylformamide, N-methylpyrrolidone, dimethyl sulfoxide, cyclohexanone, methyl ethyl ketone and methyl isobutyl ketone; surfactants, emulsifiers or disper sants such as alkyl sulfates, alkylsulfonic acid salts, polyoxyethylene glycol ethers, polyoxyethylene alkylaryl ethers, polyoxyethylene sorbitan monoalkylates and dinaph thylmethanedisulfonic acid salts; and a variety of adjuvants such as carboxymethyl cellulose and gum arabic.

Such a formulation can be prepared, for example, by mixing the compound according to the present invention with the aforesaid carrier and/or emulsifier, etc.

The compound according to the present invention may be present in a proportion of usually 0.01 to 99% by weight, preferably 0.1 to 96 % by weight, in the formulation.

The compound according to the present invention can be applied to plants in usual methods such as spraying, atomizing, or dusting, as such or in the form of a mixture with other active compounds or in such a formulation as aforesaid.

When it is desired to inhibit the growth of, or eradicate hazardous plants, the compound according to the present invention can be applied, either as such or in the form of a composition, to the plants or seeds directly or to the soil in an amount sufficient to inhibit the growth of or eradicate the hazardous plants in a locus where useful plants or their seeds and hazardous plants or their seeds are present together or likely to grow together.

The hazardous plants may be defined as plants which come into an environment created by man, such as a paddy or an upland farm, from the surrounding nature, and grow there and which are considered by man to be useless in the environment or do harm to it. Such hazardous plants are generally called weeds. Examples of the weeds are shown below.

<u>Amaranthaceae</u>

Amaranthus viridis
Amaranthus lividus
Amaranthus retroflexas

<u>Convolvulaceae</u>

Ipomoea purpurea
Cuscuta japonica

Polygonaceae

Polygonum convolvulvs
Polygonum hydropiper
Polygonum lapathifolium
Rumex obtusifolius
Rumex crispus
Rumex bidens

Chenopodiaceae

Chenopodium album
Chenopodium album var. centrorubrum
Chenopodium ficifolium

Cruciferae

Brassica kaber
Capsella bursa-pastoris
Thlaspi arvense
Raphanus raphanistrum

Portulacaceae

Portulaca oleracea

Oeguminosae

Cassia obtusifolia
Desmodium tortuosum
Sesbania exaltata
Daubentonia texana

Asclepiadaceae

Asclepias syriaca

Labiatae

Lamium amplexicaule

Malvaceae

Abutilon theophrasti
Sida spinosa

Solanaceae

Solanum nigrum
Datula stramonium
Solanum carolinense

Plantaginaceae

Plantago lanceolata
Plantago major
Plantago rugelii

Compositae

Erigeron annuus
Ambrosia artemisiaefolia var. elator
Xanthium strumarium
Cirsium arvense var. etosum
Brdens pilosa
Helianthus sp.
Sonchus oleraceus
Matricaria chamomilla
Taraxacum officinale

Rubiaceae

Galium aparine

Gramineae

Sorghum halepense
Avena fatua
Digitaria adscendens
Setaria faberi
Agropyron repens
Panicum texanum
Echinochloa crus-galli
Setaria viridis
Poa annua
Eleusine indica
Axonopus affinis
Bachiaria platyphylla
Bromus tectorum
Cynodon dactylon
Panicum dichotomiflorum
Paspalum dilatatum
Echinochloa colona
Panicum capillare
Setaria faberi
Alopecurus acqualis var. amurensis
Dactyloctenium
Imperata cylindrica
Pennisetum alopecuroides
Alopecurus myosuroides
Bromus secalinus
Agrostis alba
Apera spica-venti
Avena ludoviciana
Bromus sterilis
Brachiaria eruciformis
Cenchrus pauciflorus
Digitaria ischaemum

Caryophyllaceae

Stellaria media

Euphorbiaceae

Euphorbia sp.

Scrophulariaceae

Veronica didyma

Cyperaceae

Cyperus rotundus
Ceperus microiria
Cyperus serotinus
Scirpus hotarui
Eleocharis acicularis var. longiseta

Alismataceae

Sagittaria pygmaea

Pontederiaceae

Monochoria vaginalis

Moreover, the compounds of formula (I) also can be used as a selective herbicide which can eradicate both broad-leaved weeds and narrow-leaved weeds substantially without chemical injury to useful crops, especially soybeans and corns, accordingly without growth inhibition of these plants, by selecting the application methods, treating methods and application rates.

For example, the compounds of the invention or compositions containing the compounds of the invention can be used as a selective herbicide which can not only eradicate the target weeds or inhibit their growth by foliar application, but also can inhibit germination and growth of the weeds by applying them before germination, substantially without inhibition of germination and growth of useful crops.

The compounds of formula (I) provided by this invention may be used in combination with various herbicidal compounds known per se. At this time, the compounds of formula (I) according to the invention are combined with another comounds showing excellent herbicidal activity against narrow-leaved weeds so that it may manifest satisfactorily herbicidal effects against both broad-leaved weeds and narrow-leaved weeds. Thus, there can be obtained a herbicidal composition which is effective against both broad-leaved weeds and narrow-leaved weeds.

Thus, the present invention provides a herbicidal composition comprising, as a herbicidal ingredient, a combination of the compound of formula (I) and an N-phosphonomethylglycine derivative of formula (IV)

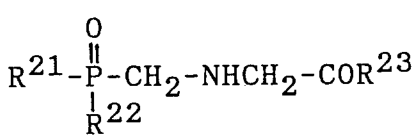

(IV)

wherein

$R^{21}$ and $R^{22}$ are identical or different and each represents a hydroxyl or $-OR^{24}$,

$R^{23}$ represents a hydroxyl , $-OR^{24}$ or $-NR^{25}R^{26}$, wherein

$R^{24}$ represents an alkyl group having 1 to 5 carbon atoms, a cyclohexyl group, a haloalkyl group having 1 to 5 carbon atoms, an alkenyl group having 2 to 5 carbon atoms or an alkoxyalkyl group or a haloalkoxyalkyl group or an alkoxyalkoxyalkyl group (wherein each of the alkoxy, haloalkoxy, and alkyl groups has 1 to 5 carbon atoms), and a phenoxy group;

$R^{25}$ and $R^{26}$ are identical or different and each represents a hydrogen atom, an alkyl group having 1 to 5 carbon atoms, a hydroxyalkyl group having 1 to 5 carbon atoms, an alkenyl group having 2 to 5 carbon atoms, or $R^{25}$ and $R^{26}$, together with the nitrogen atom to which they are attached, may form a morpholino group, a piperidino group or a pyrrolidino group,

and/or a glufosinate compound of formula (V)

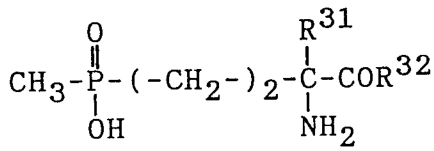

(V)

wherein

$R^{31}$ is a hydrogen atom or an alkyl group having 1 to 4 carbon atoms,

$R^{32}$ is -OH, $-NH_2$, $-NHNH_2$, $-NHC_6H_5$ or an alkoxy group having 1 to 12 carbon atoms which may be substituted by -OH,

or its acid addition salt or a salt thereof with a base, additionally a carrier and/or a surfactant.

In the formula (IV), $R^{21}$ and $R^{22}$ are identical or different and each represents -OH, or $-OR^{24}$,

$R^{23}$ represents -OH, $-OR^{24}$ or $-NR^{25}R^{26}$,

wherein

$R^{24}$ represents an alkyl group having 1 to 5 carbon atoms, a cyclohexyl group, a haloalkyl group having 1 to 5 carbon atoms, an alkenyl group having 2 to 5 carbon atoms or an alkoxyalkyl, haloalkoxyalkyl or alkoxyalkoxyalkyl group (wherein each of the alkoxy, haloalkoxy, and alkyl groups has 1 to 5 carbon atoms), and a phenoxy group,

$R^{25}$ and $R^{26}$ are identical or different and each represents a hydrogen atom, an alkyl group having 1 to 5 carbon atoms, a hydroxyalkyl group having 1 to 5 carbon atoms, and an alkenyl group having 2 to 5 carbon atoms, or

$R^{25}$ and $R^{26}$, together with the nitrogen atom to which they are attached, may form a morpholino group, a piperidino or a pyrrolidino group.

Examples of the alkyl group for $R^{24}$, $R^{25}$ and $R^{26}$ may be the same as those given hereinabove with regard to X and Y n formula (I).

Examples of the haloalkyl group having 1 to 5 carbon atoms for $R^{24}$ include halomethyl, haloethyl, dihaloethyl, halopropyl, halobutyl, and halopentyl groups. The halogen atoms may be, for example, fluorine, chlorine or bromine.

Examples of the alkenyl group having 2 to 5 carbon atoms for $R^{24}$, $R^{25}$ and $R^{26}$ include vinyl, propenyl, butenyl and pentenyl groups.

Examples of preferred alkoxyalkyl groups for $R^{24}$ are methoxymethyl and ethoxyethyl groups.

Examples of preferred haloalkoxyalkyl groups for $R^{24}$ are chlorethoxyethyl and chloromethoxyethyl groups.

Examples of preferred alkoxyalkoxyalkyl groups for $R^{24}$ are methoxyethoxyethyl and ethoxyethoxyethyl groups.

Examples of preferred hydroxyalkyl groups having 1 to 5 carbon atoms for $R^{25}$ and $R^{26}$ are hydroxymethyl, hydroxyethyl and hydroxypentyl groups.

Compounds of formula (IV) are disclosed in the Japanese Patent Published Specification No.6401/-(1981)(U.S. Patent No. 7,123,057) and are known per se.

The compound of formula (IV) also may be used in the form of its acid addition salt or its salt with a base in the composition of this invention.

Strong acids with a pKa of, for example, not more than 2.5 are preferred as acids for forming the acid adducts. As such an acid, are cited hydrochloric acid, sulfuric acid, phosphoric acid, trifluoroacetic acid, and trichloroacetic acid.

The salt with a base is formed, for example, as a salt with a cation such as an alkali metal, an alkaline earth metal, copper, zinc, ammonium or an organic ammonium or trimethylsulfonium, triethylsulfonium, tripropylsulfonium, trimethylsulfoxonium, triethylsulfoxonium, tripropylsulfoxonium, when at least one of $R^{21}$, $R^{22}$ and $R^{23}$ represent -OH.

The alkali metal represents, for example, lithium, sodium and potassium, and the alkaline earth metal represents, for example, magnesium and calcium.

The organic ammonium salt is produced from an organic amine having a low molecular weight of less than about 300.

Examples of the organic amine include alkylamines, alkylenepolyamines, and alkanolamines, such as methylamine, ethylamine, n-propylamine, isopropylamine, n-butylamine, isobutylamine, secondary butylamine, n-amylamine, isoamylamine, hexylamine, heptylamine, octylamine, nonylamine, decylamine, undecylamine, dodecylamine, tridecylamine, tetradecylamine, pentadecylamine hexadecylamine, heptadecylamine, octadecylamine, methylethylamine, methylisopropylamine, methylhexylamine, methylnonylamine, methylpentadecylamine, methyloctadecylamine, ethylbutylamine, ethylheptylamine, ethyloctylamine, hexylheptylamine, hexyloctylamine, dimethylamine, diethylamine, di-n-propylamine, diisopropylamine, di-n-amylamine, di-isoamylamine, dihexylamine, diheptylamine, dioctylamine, trimethylamine, triethylamine, tri-n-propylamine, tri-isopropylamine, tri-n-butylamine, tri-isobutylamine, tri-secondary butylamine, tri-n-amylamine, ethanolamine, n-propanolamine, isopropanolamine, diethanolamine, N,N-diethylethanolamine, N-ethylpropanolamine, N-butylethanolamine, allylamine, n-butenyl-2-amine, n-pentenyl-2-amine, 2,3-dimethylbutenyl-2-amine, di-butenyl-2-amine, n-hexenyl-2-amine, and propylenediamine, primary arylamines, such as aniline, methoxyaniline, ethoxyaniline, o, m, p-toluidine, phenylenediamine, 2,4,6-tribromoaniline, benzidine, naphthylamine, and o, m, p-chloroaniline; and heterocyclic amines such as pyridine, morpholine, piperidine, pyrrolidine, indoline, and azepine.

Preferred compounds of formula (IV) are those in which one or two of $R^{21}$, $R^{22}$, and $R^{23}$ are -OH, -OH salt or $-OR^{24}$ and the remain der of $R^{21}$, $R^{22}$ and $R^{23}$ is -OH. Examples of the salts of -OH are ammonium or organic ammonium salts in which the organic ammonium group is selected from monoalkylammonium, dialkylammonium, trialkylammonium, monoalkenylammonium, dialkenylammonium, trialkenylammonium, monoalkynylammonium, dialkynylammonium, trialkynylammonium, monoalkanolammonium, dialkanolammonium, trialkanolammonium, heterocyclic ammonium and arylammonium, and contains 1 to 18 carbon atoms.

The salts of the compounds of formula (IV) with acids or bases can be produced by methods known per se from the compounds of formula (IV) with the acids or bases.

The following compounds of formula (IV) and their acid addition salts or their salts with bases are preferably used in the present invention:

(101) N-phosphonomethylglycine.
(102) N-phosphonodimethylglycine sodium salt.
(103) N-phosphonomethylglycine ammonium salt.
(104) N-phosphonomethylglycine calcium salt monohydrate.
(105) N-phosphonomethylglycine magnesium salt.
(106) N-phosphonomethylglycine potassium salt.
(107) N-phosphonomethylglycine dimethylammonium salt.
(108) N-phosphonomethylglycine copper salt.
(109) N-phosphonomethylglycine zinc salt.
(110) N-phosphonomethylglycinamide.
(111) methyl N-phosphonomethylglycinate.
(112) ethyl N-phosphonomethylglycinate.
(113) n-propyl N-phosphonomethylglycinate.
(114) n-butyl N-phosphonomethylglycinate.
(115) cyclohexyl N-phosphonomethylglycinate.
(116) chloroethyl N-phosphonomethylglycinate.
(117) N-phosphonomethylglycine isopropylammonium salt.
(118) N-phosphonomethylglycine methylammonium salt.
(119) N-phosphonomethylglycine diisopropylammonium salt.
(120) N-phosphonomethylglycine pyridinium salt.
(121) N-phosphonomethylglycine anilinium salt.
(122) N-phosphonomethylglycine trimethylsulfonium salt.
(123) N-phosphonomethylglycine trimethylsulfoxonium salt.

In the formula (V), $R^{31}$ is a hydrogen atom or an alkyl group having 1 to 4 carbon atoms,

$R^{32}$ is -OH, $-NH_2$, $-NHNH_2$, $-NHC_6H_5$ or an alkoxy group having 1 to 12 carbon atoms which may be substituted by -OH.

Examples of the alkyl group for $R^{31}$ may be alkyl groups having 1 to 4 carbon atoms among the alkyl groups exemplified for X and Y in formula (I).

The alkoxy group for $R^{32}$ may be straight-chained or branched, and includes, for example, methoxy, ethoxy, n-propoxy, iso-propoxy, n-butoxy, sec-butoxy, iso-butoxy, tert-butoxy, n-pentoxy, n-hexoxy, n-heptoxy, n-octoxy, n-nonanoxy, n-decanoxy, n-undecanoxy, and dodecanoxy groups. These alkoxy groups may be substituted by -OH, thus, for example, $R^{32}$ may represent a hydroxyethoxy group.

Compound of formula (V) are disclosed in Japanese Patent Publication No. 26564/1982 (U. S. Patent No. 4,168,963) and believed to be known.

The compound of formula (V) may be used in the composition of this invention, as an acid addition salt or a salt with a base.

Examples of acids for forming the acid addition salts may be those exemplified hereinabove with regard to the acid addition salts of the compound of formula (IV). It is believed that the acid addition salts are formed at the primary amino group in formula (V).

Examples of the bases for forming the salt may be those exemplified above with regard to the compounds of formula (IV).

In the formula (V), $R^{31}$ is preferably a hydrogen atom and $R^{32}$ is preferably -OH, $-NH_2$, $-NHNH_2$, an alkoxy group having 1 to 4 carbon atoms, and a hydroxyalkoxy group having 2 to 4 carbon atoms.

Examples of preferred salts of the compounds of formula (V) include salts with Na, K, Cu, Mg, Ca, Zn, Ni, Mn or Fe, ammonium salts, salts with bases such as mono-, di-, or trialkylamine having 1 to 4 carbon atoms in each alkyl moiety, or aniline, acid adducts with acids such as hydrochloric acid, sulfuric acid, hydrobromic acid, chloric acid, or oxalic acid.

The salts of the compounds of formula (V) with the acids or bases may be produced by known methods from the compounds of formula (V) and the acids or bases.

Examples of the compounds of formula (V) and their acid adducts or their salts with bases which are preferably used in this invention are shown below.

(500) [(3-amino-3-carboxy)-propyl-1]-methyl-phosphinic acid
(501) [(3-amino-3-carboxy)-propyl-1]-methyl-phosphinic acid monosodium salt.
(502) [(3-amino-3-carboxy)-propyl-1]-methyl-phosphinic acid monopotassium salt.
(503) [(3-amino-3-carboxy)-propyl-1]-methyl-phosphinic acid monoammonium salt.
(504) [(3-amino-3-carboxy)-propyl-1]-methyl-phosphinic acid diammonium salt.
(505) [(3-amino-3-carboxy)-propyl-1]-methyl-phosphinic acid magnesium salt.
(506) [(3-amino-3-carboxy)-propyl-1]-methyl-phosphinic acid monopropylammonium salt.
(507) [(3-amino-3-carboxy)-propyl-1]-methyl-phosphinic acid mono(diisopropylammonium) salt.
(508) [(3-amino-3-carbomethoxy)-propyl-1]-methyl-phosphinic acid.
(509) [(3-amino-3-carbomethoxy)-propyl-1]-methyl-phosphinic acid sodium salt.
(510) [(3-amino-3-carbomethoxy)-propyl-1]-methyl-phosphinic acid diisopropylammonium salt.
(511) [(3-amino-3-carbamido)-propyl-1]-methyl-phosphinic acid.
(512) [(3-amino-3-carbamido)-propyl-1]-methyl-phosphinic acid sodium salt.
(513) [(3-amino-3-carbamido)-propyl-1]-methyl-phosphinic acid ammonium salt.
(514) [(3-amino-3-methyl-3-carboxy)-propyl-1]-methyl-phosphinic acid.
(515) [(3-amino-3-methyl-3-carboxy)-propyl-1]-methyl-phosphinic acid monosodium salt.
(516) [(3-amino-3-methyl-3-carboxy)-propyl-1]-methyl-phosphinic acid monoammonium salt.

Bialaphos also may be used as a similar compound of formula (V) for mixing with the compound of formula (V).

Further, a triazine herbicide (simazine, atrazine, cyanazine, simetryn, prometrin or metribuzin) can be mixed with the compound of formula (I) of the invention in addition to the compounds of formulas (IV) and (V).

The composition according to the present invention contains the compound of formula (I) and other herbicides in a (I) : the other compounds weight ratio of from 1 : 500 to 500 : 1, more preferably from 1 : 200 to 200 :1, especially preferably from 1 : 100 to 100 : 1.

The amount of the composition to be actually applied varies depending upon many factors, for example, the type of a plant whose growth is to be controlled. Suitably, it is generally 0.01 to 10 kg/ha, preferably 0.05 to 5 kg/ha. Any one skilled in the art can easily determine the suitable proportions and amount of the composition by an ordinary standardized test without conducting many experiments.

Further, when the compound of formula (I) according to the invention is used together with a variety of compounds hich are known per se, the compound of formula I is preferably only the R-enantiomer of the diphenyl ether derivative of formula I, but may include the S-enantiomer at a weight ratio of 100 R-enantiomer : 0 S-enantiomer to 50 : 50.

The composition of this invention may be applied in the form of a composition comprising the active ingredient and a carrier of a solid or liquid diluent. The composition may also include an additive such as surfactant. Examples of such diluents, carriers and surfactants may be those which have been cited hereinabove.

The composition of this invention can be used as a usual formulation, for example, as a solution, an emusifiable concentrate, a suspension, a dust or a paste in combination with a carrier and/or a surfactant.

The composition of this invention can be prepared, for example, by mixing the compound (I) and the other herbicidal compounds and then with a carrier or other additives, and formulating the resulting mixture, or by separately preparing a composition comprising the compound (I) and another composition comprising the other herbicidal compositions, adding a carrier or the like as required, then mixing these compositions,

and formulating the mixture.

According to the present invention, there is also provided a method of eradicating weeds, which comprises applying the compound of formula (I) and the other herbicidal compounds, simultaneously or successively, to a locus where weeds are growing in an amount effective for eradicating the weeds.

In this method, the compound of formula (I) and the other herbicidal compounds may be applied simultaneously to the aforesaid locus, as the composition comprising these compounds, or as the composition of the compound (I) and the composition of the other herbicidal compounds separately prepared.

Alternatively, the composition of the compound (I) and the composition of the other herbicidal compounds, separately prepared, may be applied to the aforesaid locus successively with the passage of time. At this time, the sequence of applications of the composition of the compound (I) and the composition of the other herbicidal compounds is not limited.

After one of the compositions is applied, the other may preferably be applied, while the active compound or compounds still remain on the foliage of the weeds. Usually, after one of the composition is applied, the other may preferably be applied immediately or within 2 to 3 days, although this period may vary depending upon the type of the plant to be controlled, the climatic conditions, etc.

According to the present invention, the compound of formula (I) and the other herbicidal compounds, for example, may be applied to a locus where a crop is cultivated before emergence of the crop. As a result, weeds growing in the locus before emergence of the crop can be killed and the crop can be grown favorably.

The application amounts of the compound (I) and other herbicidal compounds are determined suitably as a measure of the amounts of these compounds described in the compositions.

Advantageously, according to the method of this invention, both broad-leaved weeds and narrow-leaved weeds can be eradicated at a low application rate.

Moreover, the compounds of formula (I) according to the present invention also can be applied in combination with a variety of pesticides such as insecticides or herbicides which are known per se.

Effects of the invention:

The use of R-enantiomer of the diphenyl ether derivaive represented by general formula (I) according to the present invention has enabled the provision of a herbicide of more excellent herbicidal activity with a reduced application rate than the use of the racemic isomer. Further, the combination of a diphenyl ether derivative of formula (I) with an N-phosphonomethylglycine derivative of formula (IV) and/or a glufosinate compound of formula (V) has been found that the synergisic effect is developed to increase the quick-acting properties and remarkable expansion of the herbicidal spectrum with low application rate in comparison with single use of N-phosphonomethylglycine derivative or of a gluphosinate compound.

Examples

The following examples illustrate the present invention in greater detail.

In these examples, all parts means parts by weight, unless otherwise noted. The herbicidal activity was evaluated on a scale of 6 degrees. In other words, 0 means the state where the plants are as sound as before the application of the active compound, while 5 is the state where the plants are withered or killed by application of the active compound, and 1, 2, 3 and 4 mean varying degrees of the enfeebled state of the plants between 0 and 5.

Example 1

Synthesis of R-enantiomer of methyl α-[2-cyano-5-(2-chloro-4-trifluoromethylphenoxy)phenoxy]propionate

4-(2-Chloro-4-trifluoromethylphenoxy)-2-hydroxybenzaldehyde (6.3 parts), hydroxylamine hydrochloride (1.8 part), sodium formate (2.5 parts) and formic acid (30 parts by volume) were mixed and heated under reflux for 1 hour, then poured into ice-water. The mixture was extracted with ethyl ether, and the organic phase was washed with water, then dried over anhydrous sodium sulfate. Ethyl ether was distilled off under reduced pressure and the residue was recrystallized from benzene and n-hexane to give 4-(2-chloro-4-trifluoromethylphenoxy)-2-hydroxybenzonitrile (5.9 parts) (melting point: 162 to 164 °C; the IR and NMR spectral data are shown in Table 1).

The 4-(2-chloro-4-trifluoromethylphenoxy)-2-hydroxybenzonitrile (9.4 parts), S-enantiomer of methyl o-(p-toluenesulfonyl)lactate (7.8 parts), anhydrous potassium carbonate (8.4 parts), and acetonitril (50 parts by volume) were mixed and heated under reflux for 8 hours, poured into ice-water, and extracted with ethyl ether. The ethyl ether layer was dried over anhydrous sodium sulfate, then evaporated under reduced pressure and the residue was chromatographed over the silica-gel column to give the target R-enantiomer (11.6 parts). The IR and NMR spectral data and specific rotation of the compound are shown in Table 1. Further, said compound was obtained by using a prescribed amount of the S-enantiomer of methyl o-methanesulfonyllactate instead of the S-enantiomer of methyl o-(p-toluenesulfonyl)lactate, too.

Example 2

Synthesis of R-enantiomer of methyl α-[2-cyano-5-(2-chloro-4-trifluoromethyl-phenoxy)phenoxy]propionate [compound (1)]

4-(2-Chloro-4-trifluoromethylphenoxy)-2-hydroxybenzaldehyde (1.0 part) was dissolved in methanol (3 parts by volume) and the solution was poured into methaol (5 parts by volume) saturated with ammonia. The solution was combined with a sodium mothoxide (0.34 part) solution in methanol (5 parts by volume), and iodine (0.4 parts) dissolved in methanol (5 parts by volume) was added to the solution. After the mixture was stirred at room temperature for 2 hours, methaol was removed under reduced pressure, the residue was combined, acidified with HCl, then extracted with ethyl ether. The ether phase was dried over anhydrous sodium sulfate, evaporated under reduced pressure and the crude product was purified by silica-gel column chromatography to give the target compound, 4-(2-chloro-4-trifluoromethylphenoxy)-2-hydroxybenznitrile (0.7 part).

The product was allowed to react with S-enantiomer of o-(p-toluenesulfonyl)lactic ester (0.58 parts) in the same manner as in Example 1, to give the target compound (0.85 part).

Example 3

Synthesis of R-enantiomer of ethyl α-[2-cyano-5-(2-chloro-4-trifluoromethylphenoxy)phenoxy]propionate [compound (2)]

(R)-2-(1-carboethoxy)-4-(2-chloro-4-trifluoromethylphenoxy)benzaldehyde (7.77 parts), hydroxylamine hydrochloride (1.8 part), sodium formate (2.5 parts), and formic acid (30 parts by volume) were mixed, heated under reflux for 1 hour, and poured into ice-water. The mixture was extracted with ethyl ether, the organic phase was washed with water and dried over anhydrous sodium sulfate. Ethyl ether was evaporated under reduced pressure, the residue was combined with thionyl chloride (5 parts), stirred at 45°C for 3 hours, then the excess thionyl chloride was removed under reduced pressure. The residue was mixed with ethanol (30 parts by volume) and triethylamine (2.1 parts) was added dropwise to the mixture at room temperature. The reaction mixture was poured into ice-water, extracted with ethyl ether. The ethyl ether phase was dried over anhydrous sodium sulfate, the solvent was removed under reduced pressure, and the residue was purified by silica-gel column chromatography to give the target substance (6.2 parts).

The NMR and IR spectral data and specific rotation of aid compound are shown in Table 1.

Example 4

Synthesis of R-enantiomer of n-butyl α-[2-cyano-5-(2-chloro-4-trifluoromethylphenoxy)phenoxy]propionate [compound (3)]

Example 3 was repeated except that using n-butanol instead of ethanol to give the target substance (5.5 parts).

The NMR and IR spectral data and specific rotation of said compound are shown in Table 1.

Example 5

Synthesis of R-enantiomer of ethyl α-[2-cyano-5-(3-chloro-5-trifluoromethyl-2-pyridyloxy)phenoxy]propionate [compound (37)]

Example 1 was repeated except using 4-(3-chloro-5-trifluoromethyl-2-pyridyloxy)-2-hydroxybenzaldehyde instead of 4-(2-chloro-4-trifluoromethylphenoxy)-2-hydroxy-benzaldehyde and S-enantiomer of ethyl o-methanesulfonyllactate (5.9 parts) instead of S-enantiomer of methyl o-(p-toluenesulfonyl)lactate (7.8 parts) to give the target substance (6.0 parts).

The NMR and IR spectral data and specific rotation of said compound are shown in Table 1.

Comparative Example 1

Synthesis of racemic methyl α-[2-cyano-5-(2-chloro-4-trifluoromethylphenoxy)phenoxy]propionate [compound (1)]

Example 1 was repeated except using methyl DL-2-bromo-propionate (5.0 parts) instead of the S-enantiomer of methyl toluenesulfonyl)lactate (7.8 parts) to give the target substance (10.5 parts).

The NMR and IR spectral data and specific rotation of said compound are shown in Table 1.

Comparative Example 2

Synthesis of S-enantiomer of methyl α-[2-cyano-5-(2-chloro-4-trifluoromethylphenoxy)phenoxy]propionate [compound (1)]

Example 1 was repeated except using R-enantiomer of methyl o-(p-toluenesulfonyl)lactate instead of the S-enantiomer to give the target S-enantiomer (11.5 parts).

The NMR and IR spectral data and specific rotation of said compound are shown in Table 1.

Table 1

| Example No. | Compound No. | IR Spectra ($cm^{-1}$) | NMR spectra ($\delta$ ppm, $CCl_4$) | Specific Rotation $[\alpha]_D^{25}$ in THF |
|---|---|---|---|---|
| 1 | (1) | 2210<br>1755<br>1600<br>1490<br>1430<br>1320 | 1.70(3H), 3.70(3H),<br>4.70(1H), 6.35(1H),<br>6.47(1H), 7.13(1H),<br>7.45(1H), 7.55(1H),<br>7.75(1H) | +20.2 (c = 2.62) |
| 3 | (2) | 2210<br>1750<br>1590<br>1490<br>1420<br>1320 | 1.20(3H), 1.67(3H),<br>4.10(2H), 4.63(1H),<br>6.30(1H), 6.43(1H),<br>7.10(1H), 7.45(1H),<br>7.55(1H), 7.70(1H), | +14.2 (c = 0.51) |
| 4 | (3) | 2210<br>1745<br>1590<br>1485<br>1420<br>1320 | 0.7-1.9(7H),<br>1.65(3H), 4.07(2H),<br>4.67(1H), 6.40(1H),<br>6.45(1H), 7.10(1H),<br>7.45(1H), 7.55(1H),<br>7.70(1H) | +11.3 (C = 0.54) |
| 5 | (37) | 2210<br>1640<br>1590<br>1490<br>1450<br>1420<br>1320 | 1.20(3H), 1.70(3H),<br>4.17(2H), 4.70(1H),<br>6.70(1H), 6.80(1H),<br>7.56(1H), 7.97(1H),<br>8.20(1H) | +18.2 (c = 0.52) |

Table 1 (continued)

| Example No. | Compound No. | IR Spectra ($cm^{-1}$) | NMR spectra ( δ ppm, $CCl_4$) | Specific Rotation $[\alpha]_D^{25}$ in THF |
|---|---|---|---|---|
| Compa. 1 | (1) racemic mixture | 2210<br>1755<br>1600<br>1490<br>1430<br>1320 | 1.70(3H), 3.70(3H), 4.70(1H), 6.35(1H), 6.47(1H), 7.13(1H), 7.45(1H), 7.55(1H), 7.75(1H) | 0.0 (c = 2.00) |
| Compa. 2 | (1) S-enantiomer | 2210<br>1755<br>1600<br>1490<br>1430<br>1320 | 1.70(3H), 3.70(3H), 4.70(1H), 6.35(1H), 6.47(1H), 7.13(1H), 7.45(1H), 7.55(1H), 7.75(1H) | -20.2 (c=2.20) |
| Reference<br>4-(2-chloro-4-trifluoromethyl-phenoxy)-2-hydroxybenzo-nitrile | | 3320<br>2220<br>1610<br>1595<br>1510<br>1490<br>1330 | 6.40(1H), 6.60(1H), 7.13(1H), 7.40(1H), 7.50(1H), 7.75(1H), 10.2-10.7(1H), | |

TEST EXAMPLES 1 TO 5 AND COMPARATIVE EXAMPLES 3 TO 5

The diphenyl ethers shown in Table 2 were added to 5000 parts of a mixture of acetone and water (1 : 1 by volume), and 2.6 parts of a nonionic surfactant (Sorpol 2680, tradename) was added to form a solution, so that the ether becomes the prescribed amount in each solution.

Seeds of plants were sown in the soil, cultivated for 2 to 3 weeks, after germination, then the plants were used for the tests.

The prepared solution was applied to these plants at the prescribed rate of application in total, then the plants were continuously cultivated for 3 weeks without applying the solution. The results are given in Table 2.

TEST EXAMPLES 6 TO 10 AND COMPARATIVE EXAMPLE 6 AND 7

In each run, the diphenyl ether derivatives and the N-phosphonomethylglycine derivatives shown in Table 3 (in the indicated mixing ratios) was dissolved in 16 parts by volume of a mixture of water and acetone (1 : 1 by volume; containing 0.05 % of a nonionic surfactant, Sorpol-2680) in the indicated mixing ratio, respectively, to prepare spray solutions.

The plants tested were grown in vinyl resin pots (diameter 10 cm) filled with soil in a green house for 2 to 3 weeks after germination from seeds or tubers.

The spray solution was applied to the plants so that the total volume sprayed became 4 cc/100 $cm^2$, and the herbicidal activity was examined.

The results are shown in Table 3.

The alphabetical symbols in Tables 2 and 3 represent the following plants, respectively:

a: Digitaria adscendens
b: Setaria viridis
c: Sorghum halepense
d: Chenopodium album var. centrorubrum
e: Amaranthus mangostanus
f: Astragalus sinicus
g: Abutilon theophrosti
h: Solanum nigrum
i: Xanthium strumarium
j: Soybean; Glycine max
k: Corn; Zea mays

Table 2

| No. | Compound No. | Rate of Application (kg/ha) | Plants | | | | | | | | | | |
|---|---|---|---|---|---|---|---|---|---|---|---|---|---|
| | | | a | b | c | d | e | f | g | h | i | j | k |
| Test Example 1 | (1) | 0.031 | 4 | 4 | 5 | 5 | 5 | 5 | 5 | 5 | 5 | 4 | 5 |
| Test Example 2 | (1) | 0.016 | 4 | 3 | 5 | 5 | 5 | 5 | 5 | 5 | 5 | 4 | 5 |
| Test Example 3 | (2) | 0.031<br>0.008 | 4<br>3 | 4<br>3 | 5<br>3 | 5<br>5 | 5<br>3 | 5<br>4 | 5<br>5 | 5<br>5 | 5<br>5 | 5<br>4 | 5<br>4 |
| Test Example 4 | (3) | 0.031<br>0.008 | 5<br>3 | 4<br>3 | 5<br>2 | 5<br>5 | 5<br>5 | 5<br>4 | 5<br>5 | 5<br>5 | 5<br>4 | 5<br>3 | 5<br>2 |
| Test Example 5 | (37) | 0.031<br>0.008 | 4<br>3 | 4<br>3 | 5<br>3 | 5<br>3 | 5<br>5 | 4<br>3 | 5<br>5 | 4<br>4 | 5<br>4 | 4<br>3 | 3<br>2 |
| Comparative Example 3 | Racemic (1) | 0.031 | 4 | 3 | 5 | 5 | 5 | 5 | 5 | 5 | 5 | 4 | 5 |
| Comparative Example 4 | Racemic (1) | 0.016 | 3 | 2 | 2 | 5 | 5 | 5 | 5 | 5 | 5 | 4 | 4 |
| Comparative Example 5 | S-enantiomer (1) | 0.031 | 1 | 0 | 1 | 1 | 0 | 1 | 1 | 2 | 0 | 1 | 0 |

Table 3

| No. | Compound No. | Application (Kg/ha) | Days after treatment | Plants | | | | | | | | | | |
|---|---|---|---|---|---|---|---|---|---|---|---|---|---|---|
| | | | | a | b | c | d | e | f | g | h | i | j | k |
| Test Ex. 6 | (1) (117) | 0.031<br>0.375 | 3<br>7<br>14 | 2<br>5<br>5 | 2<br>4<br>4 | 3<br>5<br>5 | 2<br>5<br>5 | 2<br>5<br>5 | 2<br>4<br>5 | 4<br>5<br>5 | 4<br>5<br>5 | 2<br>5<br>5 | 3<br>4<br>5 | 2<br>5<br>5 |
| Test Ex. 7 | Racemic (1) | <br>0.031 | 3<br>7 | 2<br>4 | 2<br>4 | 3<br>4 | 1<br>5 | 0<br>5 | 2<br>4 | 4<br>5 | 4<br>5 | 4<br>5 | 4<br>4 | 3<br>5 |
| | (117) | 0.375 | 14 | 4 | 4 | 5 | 5 | 5 | 5 | 5 | 5 | 5 | 4 | 5 |
| Test Ex. 8 | (2) (117) | <br>0.031<br>0.375 | 3<br>7<br>14 | 4<br>5<br>5 | 3<br>4<br>5 | 4<br>5<br>5 | 3<br>5<br>5 | 4<br>5<br>5 | 3<br>4<br>5 | 4<br>5<br>5 | 4<br>5<br>5 | 4<br>5<br>5 | 4<br>4<br>5 | 3<br>4<br>5 |
| Test EX. 9 | (3) (117) | 0.031<br>0.375 | 3<br>7<br>14 | 4<br>4<br>5 | 3<br>4<br>5 | 4<br>4<br>5 | 3<br>4<br>5 | 3<br>4<br>5 | 4<br>4<br>5 | 4<br>5<br>5 | 4<br>5<br>5 | 4<br>5<br>5 | 4<br>4<br>5 | 3<br>3<br>3 |
| Test Ex.10 | (37) (117) | 0.031<br>0.375 | 3<br>7<br>14 | 4<br>5<br>5 | 4<br>5<br>5 | 4<br>5<br>5 | 3<br>4<br>5 | 4<br>5<br>5 | 2<br>4<br>4 | 5<br>5<br>5 | 4<br>5<br>5 | 4<br>5<br>5 | 3<br>4<br>4 | 3<br>3<br>3 |
| Compa. Ex. 6 | S-enan tiomer | <br>0.031 | 3<br>7 | 0<br>1 | 0<br>0 | 1<br>1 | 0<br>1 | 0<br>0 | 1<br>1 | 1<br>2 | 0<br>1 | 1<br>1 | 1<br>1 | 0<br>1 |
| | (117) | 0.375 | 14 | 3 | 1 | 1 | 1 | 0 | 1 | 2 | 2 | 1 | 1 | 1 |
| Compa. Ex. 7 | (117) | <br>0.375 | 3<br>7<br>14 | 3<br>1<br>3 | 0<br>0<br>1 | 0<br>0<br>1 | 0<br>0<br>1 | 0<br>0<br>0 | 0<br>0<br>0 | 0<br>1<br>1 | 0<br>0<br>0 | 0<br>0<br>1 | 0<br>0<br>0 | 0<br>0<br>0 |

## TEST EXAMPLES 11

In each run, the compound (1) according to the invention only or the N-phosphonomethylglycine derivatives (117) only or the mixture thereof were used as in the Test Example 1 to prepare single solutions and mixed solutions by using various amounts of each components. Each of the preparations were applied to the plants as in Test Example 1 and the cultivation was continued for 3 weeks without additive application thereafter. The results are given in Table 4 and Table 5.

The results were analyzed by Tammes's method [Tammes, P.M.L.: Neth. J. Plant Path., 70, 73-80 (1964)] which was cited in "Zasso Kenkyuu" (in Japanese) (Weed Studies) 14, 12-18 (173) and shown in Fig. 1 and Fig. 2.

Fig. 1 represents the average withering rate of 4 kinds of narrow-leaved plants (a, b, c, and k), while Fig. 2 gives withering rate of 7 kinds of broad-leaved plants (d, e, f, g, h, i j). As these figures show clearly, the curve binding the points of 95 % effect is inside the one of the synergistic effect, thus it can be concluded that the mixed preparation has synergistic effect.

Table 4

| Average Withering Rates of Narrow-leaved Plants (a, b, c, k) | | | | | |
|---|---|---|---|---|---|
| Compound (117) (kg a.i./ha) | Compound (1) (kg a.i./ha) | | | | |
| | 0 | 0.008 | 0.016 | 0.031 | 0.063 |
| 0 | 0 | 33 | 47 | 80 | 93 |
| 0.094 | 50 | 53 | 80 | 90 | 100 |
| 0.188 | 87 | 93 | 92 | 100 | 98 |
| 0.375 | 87 | 92 | 100 | 100 | 100 |
| 0.750 | 95 | 100 | 100 | 100 | 100 |

Table 5

| Average Withering Rates of Broad-leaved Plants (d, e, f, g, h, i, j) | | | | |
|---|---|---|---|---|
| Compound (117) (kg a.i./ha) | Compound (1) (kg a.i./ha) | | | |
| | 0 | 0.008 | 0.016 | 0.031 |
| 0 | 0 | 80 | 80 | 96 |
| 0.094 | 4 | 80 | 96 | 100 |
| 0.188 | 36 | 90 | 96 | 100 |
| 0.375 | 55 | 96 | 96 | 100 |
| 0.750 | 80 | 100 | 96 | 100 |

Brief Illustration of the Drawings

Fig. 1 is the average same-effect curve of 95 % growth inhibition against narrow-leaved plants.
Fig. 2 is the average same-effect curve of 95 % growth inhibition against broad-leaved plants.

**Claims**

**1.** A R-enantiomers of diphenyl ether derivative represented by the following formula (I):

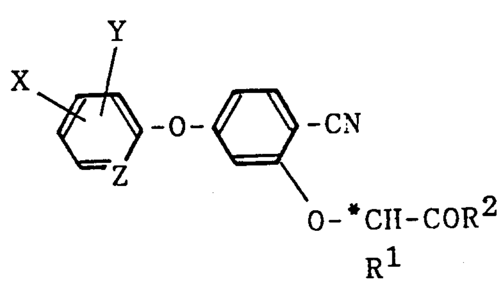

( I )

wherein

X and Y are identical or different and each represents a hydrogen atom, a halogen atom, $-CF_3$, or an alkyl group having 1 to 5 carbon atoms;

Z is =CH- or =N-;

$R^1$ is an alkyl group having 1 to 5 carbon atoms or a phenyl group;

$R^2$ is a hydroxy group, an alkyl group having 1 to 5 carbon atoms which may be optionally substituted with halogen atoms, a phenyl group which may be substituted with halogen atoms, an alkoxy group having 1 to 5 carbon atoms, an alkenyloxy group having 1 to 5 carbon atoms or

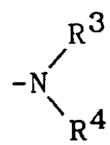

in which $R^3$ and $R^4$ are a hydrogen atom or an alkyl group having 1 to 5 carbon atoms;
C* represents an asymmetric carbon atom;
or its salt compound.

**2.** A method of producing the compounds according to claim 1 by the processes shown in the following reaction scheme,

(i)

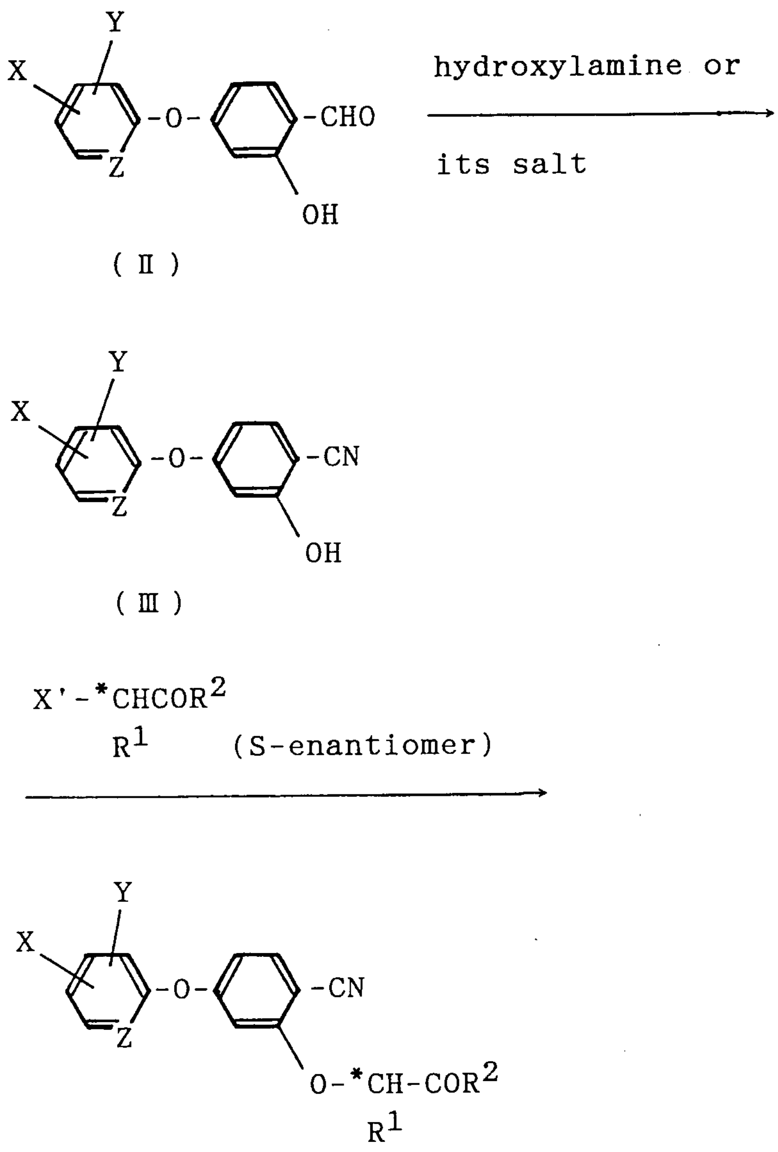


( II )

( III )

( I ) (R-enantiomer)

wherein X, Y, Z, $R^1$, $R^2$ and *C are defined as in formula (I) and
X' means a halogen atom or $-OR^5$; wherein $R^5$ is an alkylsulfonyl group or an arylsulfonyl group;

**3.** A herbicidal composition comprising a compound of claim 1 as an herbicidally active ingredient, a carrier and/or a surfactant.

**4.** A herbicidal composition comprising, as a herbicidal ingredient, a combination of the compound of formula (I) and an N-phosphonomethylglycine derivative of formula (IV)

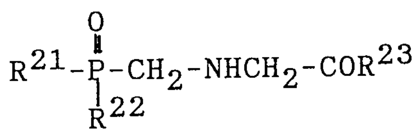

(IV)

wherein

$R^{21}$ and $R^{22}$ are identical or different and each represents a hydroxyl or $-OR^{24}$,

$R^{23}$ represents a hydroxyl , $-OR^{24}$ or $-NR^{25}R^{26}$, wherein

$R^{24}$ represents an alkyl group having 1 to 5 carbon atoms, a cyclohexyl group, a haloalkyl group having 1 to 5 carbon atoms, an alkenyl group having 2 to 5 carbon atoms, an alkoxyalkyl group or a haloalkoxyalkyl group or an alkoxyalkoxyalkyl group (wherein each of the alkoxy, haloalkoxy, and alkyl groups has 1 to 5 carbon atoms), and a phenoxy group;

$R^{25}$ and $R^{26}$ are identical or different and each represents a hydrogen atom, an alkyl group having 1 to 5 carbon atoms, a hydroxyalkyl group having 1 to 5 carbon atoms, an alkenyl group having 2 to 5 carbon atoms, or $R^{25}$ and $R^{26}$, together with the nitrogen atom to which they are attached, may form a morpholino group, a piperidino group or a pyrrolidino group,

and/or a glufosinate compound of formula (V)

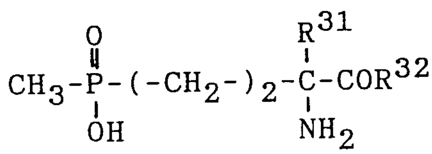

(V)

wherein

$R^{31}$ is a hydrogen atom or an alkyl group having 1 to 4 carbon atoms,

$R^{32}$ is -OH, $-NH_2$, $-NHNH_2$, $-NHC_6H_5$ or an alkoxy group having 1 to 12 carbon atoms which may be substituted by -OH,

or its acid addition salt or a salt thereof with a base, additionally a carrier and/or a surfactant.

Fig. 1: Average Withering Rates of Narrow-leaved Plants 21 Days after the Treatment

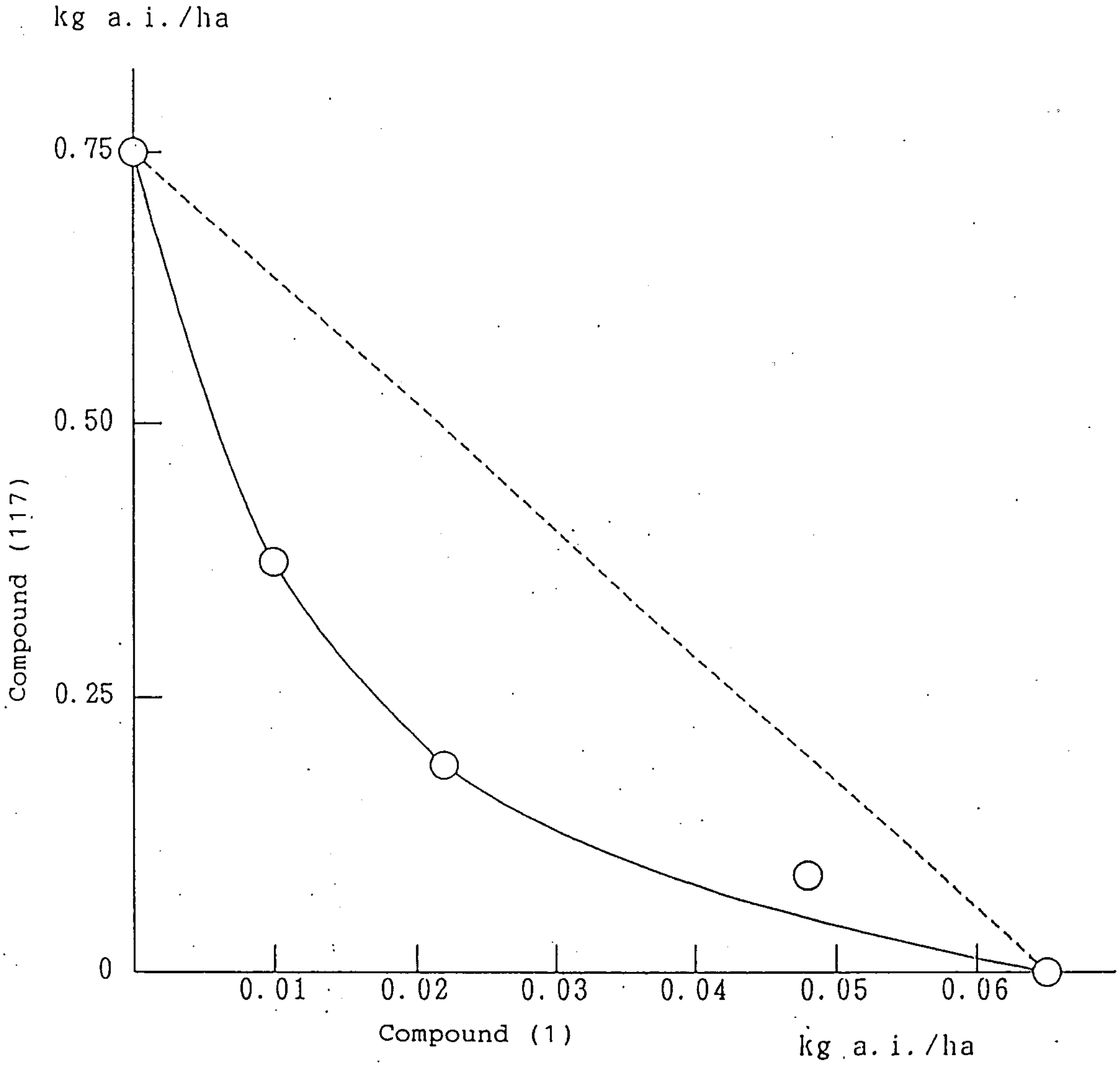

Fig. 2: Average Withering Rates of Broad-leaved Plants 21 Days after the Treatment

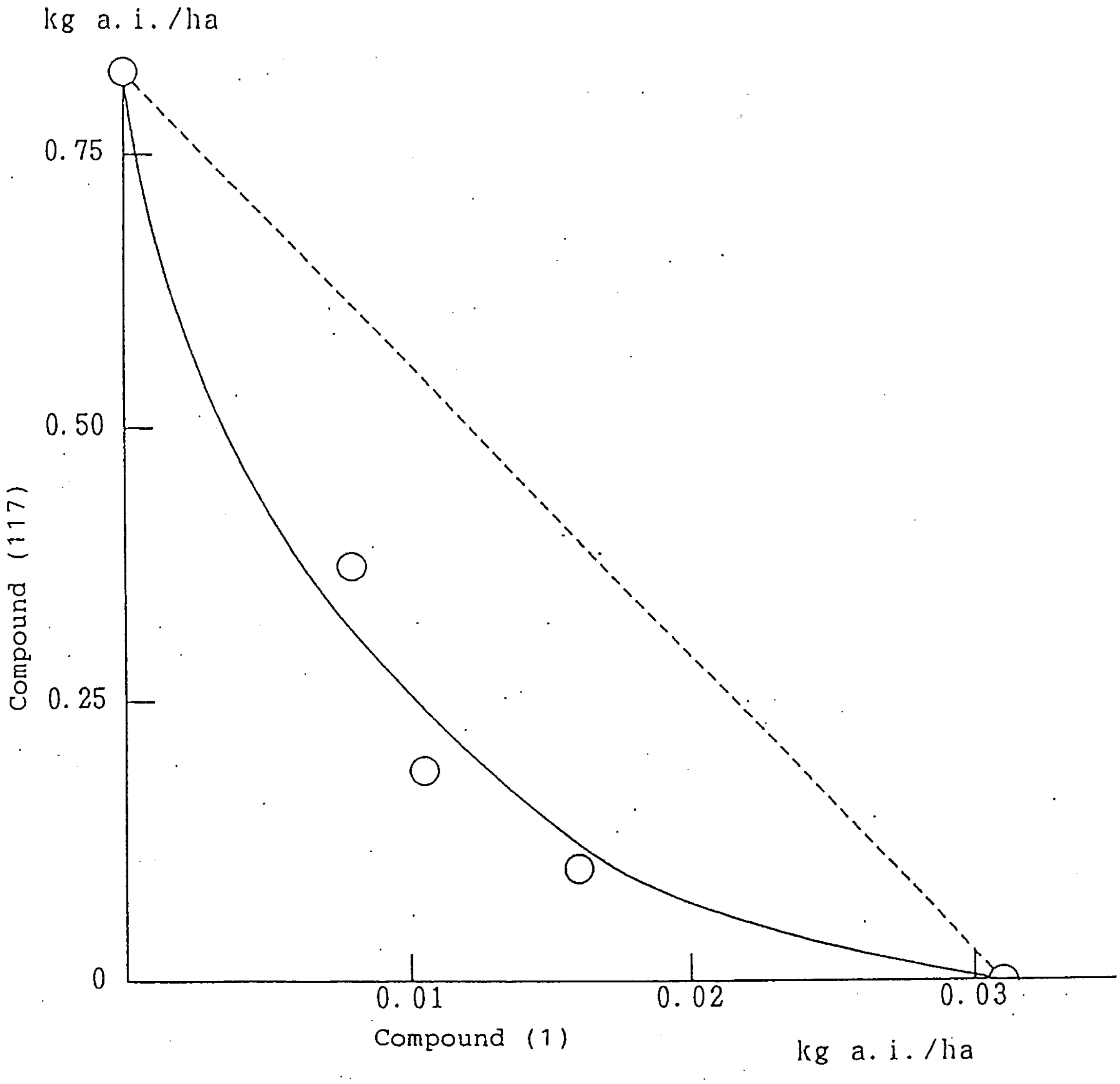

# INTERNATIONAL SEARCH REPORT

International Application No PCT/JP91/00714

**I. CLASSIFICATION OF SUBJECT MATTER** (if several classification symbols apply, indicate all) [6]

According to International Patent Classification (IPC) or to both National Classification and IPC

Int. $Cl^5$ C07C255/54, 253/02, 253/30, C07D213/643, A01N39/02, 43/40

**II. FIELDS SEARCHED**

Minimum Documentation Searched [7]

| Classification System | Classification Symbols |
|---|---|
| IPC | C07C255/54, 253/02, 253/30, C07D213/643, A01N39/02, 43/40 |

Documentation Searched other than Minimum Documentation to the Extent that such Documents are Included in the Fields Searched [8]

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| X | JP, A, 52-111528 (Ishihara Sangyo Kaisha, Ltd.), September 19, 1977 (19. 09. 77), & DE, A1, 2643438 & FR, A1, 2325638 & GB, A, 1497520 & US, A, 4070177 & AU, A1, 1790976 & NZ, A, 181994 & US, A, 4203758 & CA, A1, 1101435 | 1-3 |
| X | JP, A, 60-181048 (Byear AG), September 14, 1985 (14. 09. 85), & IL, A0, 74214 & DK, A, 48085 & EP, A2, 151429 & DE, A1, 3403974 & ZA, A, 8500793 | 1-3 |
| Y | JP, A, 52-111528 (Ishihara Sangyo Kaisha, Ltd.), September 19, 1977 (19. 09. 77), & DE, A1, 2643438 & FR, A1, 2325638 & GB, A, 1497520 & US, A, 4070177 & AU, A1, 1790976 & NZ, A, 181994 & US, A, 4203758 & CA, A1, 1101435 | 4 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| August 1, 1991 (01. 08. 91) | August 26, 1991 (26. 08. 91) |
| **International Searching Authority** | **Signature of Authorized Officer** |
| Japanese Patent Office | |

**FURTHER INFORMATION CONTINUED FROM THE SECOND SHEET**

| | | |
|---|---|---|
| Y | JP, A, 60-181048 (Byear AG), September 14, 1985 (14. 09. 85), & IL, A0, 74214 & DK, A, 48085 & EP, A2, 151429 & DE, A1, 3403974 & ZA, A, 8500793 | 4 |
| Y | JP, A, 1-163152 (Teijin Ltd.), June 27, 1989 (27. 06. 89) | 4 |

**V.☐ OBSERVATIONS WHERE CERTAIN CLAIMS WERE FOUND UNSEARCHABLE [1]**

This international search report has not been established in respect of certain claims under Article 17(2) (a) for the following reasons:

1.☐ Claim numbers , because they relate to subject matter not required to be searched by this Authority, namely:

2.☐ Claim numbers , because they relate to parts of the international application that do not comply with the prescribed requirements to such an extent that no meaningful international search can be carried out, specifically:

3.☐ Claim numbers , because they are dependent claims and are not drafted in accordance with the second and third sentences of PCT Rule 6.4(a).

**VI.☐ OBSERVATIONS WHERE UNITY OF INVENTION IS LACKING [2]**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.